# EUROPEAN PATENT SPECIFICATION

(11) **EP 0 520 032 B1**
(45) Date of publication and mention of the grant of the patent: **16.09.1998**
(21) Application number: 91907118.3
(22) Date of filing: 13.03.1991
(51) Int. Cl.: A61K 31/445

(54) **USE OF SPIPERONE AS AN IMMUNOSUPPRESSANT AND ANTI-INFLAMMATORY AGENT**
VERWENDUNG VON SPIPERON ALS IMMUNSUPPRESSIVUM UND ANTIINFLAMMATORISCHES MITTEL
UTILISATION DE SPIPERONE COMME AGENT IMMUNOSUPPRESSEUR ET ANTI-INFLAMMATOIRE

(30) Priority: 16.03.1990 US 494740
(43) Date of publication of application: 30.12.1992
(73) Proprietor: Beth Israel Hospital Association, Boston, Massachusetts 02115 (US)
(72) Inventor: SHARPE, Richard, J., Newtonville, MA 02160 (US); ARNDT, Kenneth, A., Newton Centre, MA 02159 (US); GALLI, Stephen, J., Winchester, MA 01890 (US)
(74) Representative: Bassett, Richard Simon (GB)
(86) International application number: PCT/US91/01684
(87) International publication number: WO 91/13622

(56) References cited:
- WO-A-91/02527
- FR-A- 2 163 717
- US-A- 3 155 669
- US-A- 3 155 670
- US-A- 3 161 644
- US-A- 3 238 216
- US-A- 4 555 504
- BRITISH JOURNAL OF PHARMACOLOGY vol. 58, no. 3, 1976, page 445P J.F. BURKA ET AL. 'DOPAMINERGIC (CO-MEDIATOR) MODULATION OF RELEASE OF HISTAMINE AND SRS-A IN THE CALF'
- RESEARCH COMMUNICATIONS IN CHEMICAL PATHOLOGY AND PHARMACOLOGY vol. 22, no. 3, 1978, pages 447 - 453 P. EYRE 'DOPAMINE POTENTIATES ANAPHYLACTIC CONTRACTION OF PULMONARY VEIN OF CALF'
- FED. PROC. vol. 44, no. 5, 1985, USA page 7526 E.M. STERNBERGER ET AL. 'MACROPHAGE ACTIVATION BY SEROTONIN (5-HT) IS AFFECTED BY INTERFERON CONCENTRATION AND IS ANTAGONIZED BY SPIPERONE AND KETANSERIN'
- THE JOURNAL OF IMMUNOLOGY vol. 142, no. 9, 1989, pages 3171 - 3179 J-C. AMEISEN ET AL. 'A NEW INTERPRETATION OF THE INVOLVEMENT OF SEROTONIN IN DELAYED-TYPE HYPERSENSITIVITY'
- THE JOURNAL OF IMMUNOLOGY vol. 139, no. 3, 1987, pages 869 - 875 K. HELLSTRAND ET AL. 'ROLE OF SEROTONIN IN THE REGULATION OF HUMAN NATURAL KILLER CELL CYTOTOXICITY'
- LIFE SCIENCES vol. 28, no. 9, 1981, pages 1015 - 1022 J.E. LEYSEN ET AL. 'RECEPTOR BINDING OF R 41 468, A NOVEL ANTAGONIST AT 5-HT2 RECEPTORS'
- J INVEST DERMATOL vol. 99, 1992, pages 594-600 SHARPE, R.J., et al.

## Description

### Background of the Invention

The field of the invention is the suppression of immune and inflammatory responses.

Although the immunological and inflammatory responses play vital roles in the protection of animals from infections and other externally-caused diseases, these responses can at times and under certain conditions cause undesirable pathological reactions with significant associated morbidity. Examples of such reactions include host rejection of foreign organ or tissue transplants; graft-vs-host disease in which donor immunological cells present in the graft attack host tissues; diseases with proven or possible autoimmune components, such as rheumatoid arthritis, juvenile rheumatoid arthritis, psoriatic arthritis, psoriasis, leprosy reversal reactions, erythema nodosum leprosum, autoimmune uveitis, multiple sclerosis, allergic encephalomyelitis, systemic lupus erythematosis, acute necrotizing hemorrhagic encephalopathy, idiopathic bilateral progressive sensorineural hearing loss, aplastic anemia, pure red cell anemia, idiopathic thrombocytopenia, polychondritis, scleroderma, Wegener granulamatosis, chronic active hepatitis, myasthenia gravis, Steven-Johnson syndrome, idiopathic sprue, Crohn's disease, Graves opthalmopathy, sarcoidosis, primary biliary cirrhosis, primary juvenile diabetes, uveitis posterior, and interstitial lung fibrosis; allergic asthma; inappropriate allergic responses to environmental stimuli such as poison ivy, pollen, insect stings and certain foods; and inflammation resulting from any of the above immune reactions as well as from reaction to a physical agent such as occurs in sunburn or thermal, electrical, or chemical burns. Various therapeutics which have been utilized as immunosuppressants and anti-inflammatory agents include steroid hormones, anti-metabolites such as methotrexate and azathroprine, alkylating agents such as cyclophosamide and busulfan, and antibiotics.

WO91/02527, available under Article 54(3) EPC, discloses use of spiperone for topical administration for immunosuppression at the site of administration.

British Journal of Pharmacology vol. 58, no. 3, 1976, page 445, J.F. Burka *et al;* Research Communications in Chemical Pathology and Pharmacology, vol. 22, no. 3, 1978, page 447-453, P. Eyre; and Fed. Proc. vol. 44, no. 5, 1985, U.S.A. page 7526. E.M. Sternberger *et al*, each disclose that spiperone is a dopamine and 5-HT receptor antagonist.

### Summary of the Invention

In general, the invention features the use of spiperone or a spiperone derivative or a pharmaceutically acceptable salt of spiperone for suppressing an immune response or of treating inflammation in a mammal (such as a human, or a domestic animal kept for companionship or commercial purposes: e.g., a dog or a cat) by treating the mammal systemically with the spiperone, or salt or derivative. Such immunological or inflammatory responses may be attributable to, for example, diseases (herein termed "autoimmune diseases") with proven or possible autoimmune components, such as rheumatoid arthritis, juvenile rheumatoid arthritis, psoriatic arthritis, psoriasis, leprosy reversal reactions, erythema nodosum leprosum, autoimmune uveitis, multiple sclerosis, allergic encephalomyelitis, systemic lupus erythematosis, acute necrotizing hemorrhagic encephalopathy, idiopathic bilateral progressive sensorineural hearing loss, aplastic anemia, pure red cell anemia, idiopathic thrombocytopenia, polychondritis, scleroderma, Wegener granulamatosis, chronic active hepatitis, myasthenia gravis, Steven-Johnson syndrome, idiopathic sprue, Crohn's disease, Graves opthalmopathy, sarcoidosis, primary biliary cirrhosis, primary juvenile diabetes, uveitis posterior, or interstitial lung fibrosis, or may be attributable to an allergy (e.g., allergic asthma), or to transplantation-related immunity (e.g., graft-versus-host disease or transplant rejection by the host). Other examples of inflammmation treatable by the method of the invention include cutaneous and mucosal inflammatory responses, which may be attributable to a condition involving an immune responses arthritis of any type, atopic dermatitis or other examples of dermatitis, allergic reactions, asthma of any type, or autoimmune diseases (in addition to those listed above), or which may be of unknown etiology. The spiperone used in the method of the invention preferably has the following formula: 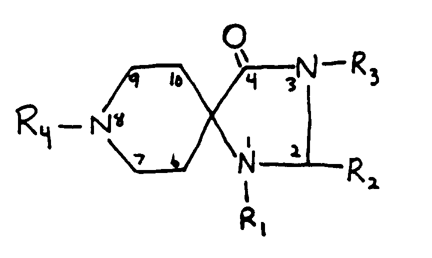 wherein
- R₁ =: H, CH₃-, C₆H₅-, cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)-, or (CH₃)₃C-;
- R₂ =: H or CH₃;
- R₃ =: H, CH₃, CH₃CH₂-, CH₃CH₂CH₂-, (CH₃)₂CH-, or CN(CH₂)₂-;
- R₄ =: H, C₆H₅CH(CH₂CH₃)CH₂-, C₆H₅CH(CH₃)(CH₂)₂-, C₆H₅CH₂(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃)(CH₂)₃, 4-X-C₆H₄CH(CH₃)CH₂-, 4-X-C₆H₄CH(CH₂CH₃)CH₂-, 4-X-C₆H₄CH(CH₃)(CH₂)₂-, 4-X-C₆H₄-CH(CH₃)(CH₂)₃-, C₆H₅CH(OCH₃)(CH₂)₂-, 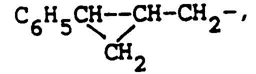 C₆H₅CO(CH₂)₃-, C₆H₅CO(CH₂)₄-, 4-(CH₃)C₆H₄CO(CH₂)₃-, 4-(CH₃O)C₆H₄CO(CH₂)₃-, 4-X-C₆H₄CO(CH₂)₃-, 4-X-C₆H₄CO(CH₂)₃-, 2-thienyl-CO(CH₂)₃-, or 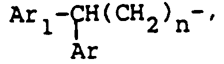
wherein n = 3 or 4; X = F, Cl, or Br; and each of Ar and Ar₁ is, independently, H, C₆H₅-, 4-(CH₃)C₆H₄-, 4-(CH₃O)C₆H₄-, 4-X-C₆H₄-, 3-(CX₃)C₆H₄-, 2-thienyl, or 4-X-C₆H₄CH₂-.
and more preferably has the following formula: 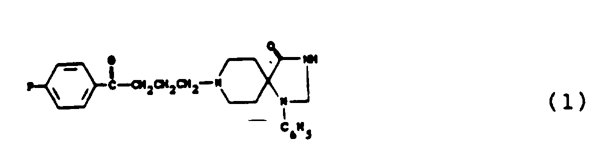 The active compound may be a CNS-blind spiperone (defined below), and/or may be combined with a pharmaceutically-acceptable diluent or carrier.

When spiperone is derivatized or complexed in such a way as to make it largely incapable of inducing significant neuropharmacological side effects such as analgesia and neurolepsia, it is termed "CNS-blind spiperone". Such a composition preferably includes spiperone combined with a cycloamylase, such as cyclodextrin; the spiperone portion of the composition preferably has the general formula for spiperone set forth above, or more preferably the formula termed "spiperone (1)".

Spiperone is a known pharmaceutical which historically has been utilized clinically as a tranquilizer useful for the treatment of schizophrenia. As such, the safety and other salient characteristics of its use in humans are well documented. Besides its previously-known neuropharmacological activities, however, spiperone has been found to have significant ability to act systemically, in the method of the invention, as a suppressor of immune and/or inflammatory responses brought about by a variety of conditions, and thus has useful applications in the treatment of these conditions. The CNS-blind spiperone of the invention is capable of suppressing such immune and inflammatory responses without the concomitant undesireable neuropharmacological side effects, such as neurolepsia, induced by spiperone when it is not in a CNS-blind form.

### Description of the Preferred Embodiments

The drawings are first described.

Fig. 1 is a bar graph illustrating the effect of systemic administration of spiperone on footpad swelling associated with IL-1-induced inflammation.

Fig. 2 is a bar graph illustrating the effect of systemic administration of spiperone on numbers of infiltrating inflammatory cells associated with IL-1-induced inflammation.

Fig. 3 is a bar graph illustrating the effect of systemic administration of spiperone on footpad swelling associated with PMA-induced inflammation.

Fig. 4 is a bar graph illustrating the effect of systemic administration of spiperone on numbers of infiltrating inflammatory cells associated with PMA-induced inflammation.

Fig. 5 is a bar graph illustrating the effect of systemic administration of spiperone on ear swelling associated with oxazolone-induced cutaneous contact hypersensitivity.

Fig. 6 is a bar graph illustrating the effect of systemic administration of spiperone on numbers of infiltrating inflammatory cells associated with oxazolone-induced cutaneous contact hypersensitivity.

### Structure and Synthesis of Spiperone

The term "spiperone" herein denotes all of the molecules which are active in the method of the invention and which are the subject of the following U.S. patents: No. 3,155,669; No. 3,155,670; No. 3,161,644; and No. 3,238,216; all of which patents are hereby incorporated by reference. Methods for the synthesis of each such compound are disclosed in said four patents.

More particularly, forms of spiperone having the following formulae may be employed in the method of the invention: 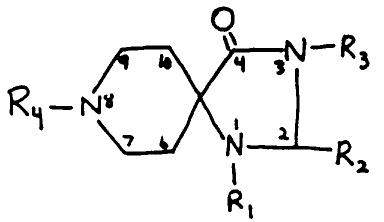 wherein
- R₁ =: H, CH₃-, C₆H₅-, cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)-, or (CH₃)₃C-;
- R₂ =: H or CH₃;
- R₃ =: H, CH₃, CH₃CH₂-, CH₃CH₂CH₂-, (CH₃)₂CH-, or CN(CH₂)₂-;
- R₄ =: H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃)(CH₂)₃, 4-X-C₆H₄CH(CH₃)CH₂-, 4-X-C₆H₄CH(CH₂CH₃)CH₂-, 4-X-C₆H₄CH(CH₃)(CH₂)₂-, 4-X-C₆H₄-CH(CH₃)(CH₂)₃-, C₆H₅CH(OCH₃)(CH₂)₂-, 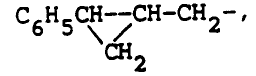 C₆H₅CO(CH₂)₃-, C₆H₅CO(CH₂)₄-, 4-(CH₃)C₆H₄CO(CH₂)₃-, 4-(CH₃O)C₆H₄CO(CH₂)₃-, 4-X-C₆H₄CO(CH₂)₃-, 4-X-C₆H₄CO(CH₂)₃-, 2-thienyl-CO(CH₂)₃-, or 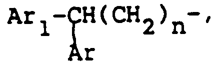
wherein n = 3 or 4; X = F, Cl, or Br; and each of Ar and Ar₁ is, independently, H, C₆H₅-, 4-(CH₃)C₆H₄-, 4-(CH₃O)C₆H₄-, 4-X-C₆H₄-, 3-(CX₃)C₆H₄-, 2-thienyl, or 4-X-C₆H₄CH₂-.

Those forms of spiperone which are particularly useful in the method of the invention include those in which R₁ is C₆H₅-, 4-(X)-C₆H₄- or 4-(CH₃)C₆H₄-; R₂ is H or CH₃; R₃ is H, CH₃, or CH₃CH₂-; and R₄ is 4-X-C₆H₄CO(CH₂)₃- or 2-thienyl-CO(CH₂)₃-; and especially those in which R₁ is C₆H₅-, 4-(Br)-C₆H₄-, or 4-(Cl)-C₆H₄-; R₂ is H or CH₃; R₃ is H, CH₃, or CH₃CH₂-; and R₄ is 4-X-C₆H₄CO(CH₂)₃- or 2-thienyl-CO(CH₂)₃-.

The particular form of spiperone used in Examples 1-3 is 8-[3-(ρ-fluorobenzoyl)propyl]-1-phenyl-1,3,8-triazaspiro[4.5]decan-4-one. Herein termed "spiperone (1)", this compound has the following structure:

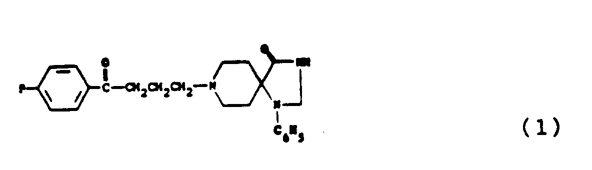

The potential utility of any one of the above-described forms of spiperone as an immunosuppressant and/or anti-inflammatory agent can be conveniently determined by synthesizing the compound and testing it in one or more of the biological assays described in Examples 1-3.

### Overcoming the Tendency of Spiperone to Affect the Central Nervous System

Spiperone has significant neuropharmacological properties, including analgesic and neuroleptic activities, which contributed to its historical use as a tranquilizer to treat schizophrenia, but which constitute generally undesirable side effects when it is employed as an immunosuppressive or anti-inflammatory agent. In an effort to increase the potency of these neuroleptic properties, Moerlein et al. (Int. J. Nucl. Med. Biol. 12:353-356, 1985) synthesized a number of forms of spiperone designed to increase the lipophilicity of the compound, and thus its potential ability to cross the blood-brain barrier. Their findings are also instructive for one selecting a compound to use in the method of the invention, for which the ability of the compound to cross the blood-brain barrier is ideally minimized. Other techniques for reducing the central nervous system (CNS) effects of spiperone include some established approaches known to the art, such as:
(a) increasing the size of the molecule via a covalent linkage to a large moiety (e.g., albumin or polyethylene glycol), using standard techniques of organic synthesis;
(b) increasing the overall hydrophilicity of the molecule by applying standard organic synthesis techniques to add one or more charged side chains onto the molecule or to alter an existing sidechain to make it more polar;
(c) a combination of (a) and (b); or
(d) forming a non-covalent complex with a cyclic molecule such as a cycloamylose (e.g., a cyclodextrin such as β-cyclodextrin), which has a spacial arrangement of hydroxyl groups whereby the outer surface of the ring formed by the cycloamylose is hydrophilic and the inner surface is lipophilic. When utilized in aqueous solution, this structure permits molecules (or parts thereof), termed "guest molecules", which are less polar than water and which are of suitable dimensions, to be incorporated into the lipophilic inner cavity, such that the cycloamylose/guest molecule complex presents to the blood-brain barrier as a relatively large and polar compound which is unable to penetrate the barrier. Such complexes may be prepared by any method known to the art, including those described in U.S. Patent No. 4,555,504, which discloses β-cyclodextrin complexed with digoxin.

Spiperone altered or complexed by any of the above methods (with the effect of reducing the CNS effects of the compound to an acceptable level), and which exhibits the ability to supress an immune and/or inflammatory response, is referred herein to as "CNS-blind spiperone". The efficacy of any particular CNS-blind spiperone entity as an immunosuppressant or anti-inflammatory agent can be conveniently tested in any of the assays described in Examples 1-3 below. Whether or nor the same entity is capable of inducing the neuropharmacological side effects observed for spiperone can be assayed by, for example, the hot plate test of Eddy et al., J. Pharmacol. 107:385(1953) and 110:135 (1954).

### Therapeutic Composition

Spiperone can be provided in the form of pharmaceutically-acceptable salts or complexes. As used herein, the term "pharmaceutically-acceptable salts or complexes" refers to salts or complexes that retain the desired biological activity of the parent compound and exhibit minimal, if any, undesired toxicological effects. Examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acids, naphthalenedisulfonic acids, and polygalacturonic acid; (b) base addition salts formed with polyvalent metal cations such as zinc, calcium, bismuth, barium, magnesium, aluminum, copper, cobalt, nickel, cadmium, and the like, or with an organic cation formed from N,N-dibenzylethylene-diamine or ethylenediamine; or (c) combinations of (a) and (b); e.g., a zinc tannate salt or the like. The composition may be in the form of a pill, tablet, capsule, or liquid for oral administration; a cream, gel, lotion, spray, or ointment for application to the skin of a patient; a liquid capable of being administered nasally as drops or spray; or a liquid capable or intravenous, subcutaneous, parenteral, or intraperitoneal administration. The therapeutic composition can also be in the form of an oil emulsion or dispersion in conjunction with a lipophilic salt such as a pamoic acid, or in the form of a biodegradable sustained-release formulation for subcutaneous or intramuscular administration. For maximum efficacy, zero-order release is desired. Zero-order release can be obtained using an implantable or external pump to administer the therapeutic composition.

### Use

Spiperone and CNS-blind spiperone are capable of acting systemically to suppress the immune and inflammatory responses in animals. As such, they, or therapeutic compositions thereof, are useful for the treatment of a myriad of immunological and/or inflammatory disorders, including those related to host rejection of foreign organ or tissue transplants; graft-vs-host disease; autoimmune diseases, such as rheumatoid arthritis, juvenile rheumatoid arthritis, psoriatic arthritis, psoriasis, autoimmune uveitis, multiple sclerosis, allergic encephalomyelitis, systemic lupus erythematosis, acute necrotizing hemorrhagic encephalopathy, idiopathic bilateral progressive sensorineural hearing loss, aplastic anemia, pure red cell anemia, idiopathic thrombocytopenia, polychondritis, scleroderma, Wegener granulamatosis, chronic active hepatitis, myasthenia gravis, Steven-Johnson syndrome, idiopathic sprue, Crohn's disease, Graves opthalmopathy, sarcoidosis, primary biliary cirrhosis, primary juvenile diabetes, uveitis posterior, and interstitial lung fibrosis; allergic reactions; and inflammation resulting from sunburn or thermal, electrical, or chemical burns.

### Example 1: Inhibition of rIL-1-induced Inflammation

0.05 ml of phosphate-buffered saline ("PBS"; GIBCO, Grand Island, NY) containing 200 units of recombinant human interleukin-1 ("rIL-1"; Genzyme Corporation, Cambridge, MA; specific activity: 100,000 units/µg) was injected intradermally into the right hind footpad of each of 15 mice (C57BL/6J female mice, 6-8 weeks old: Jackson Laboratory, Bar Harbor, ME; or Balb/c female mice, 6-8 weeks old: Charles River Laboratories, Kingston Facility, Stoneridge, NY). An identical amount of PBS was injected into the left hind footpad of each mouse. At one hour after injection of the rIL-1, the right rear flank of each mouse was subcutaneously injected with one of the following: 0.1 ml of Cremophor EL (a pharmaceutical carrier, obtained from BASF Corp., NJ); 30 mg/kg of spiperone (1) (Sigma Chemical Co., St. Louis, MO) in 0.1 ml of Cremophor EL; or 150 mg/kg spiperone (1) in 0.1 ml Cremophor EL. At eight hours after rIL-1 injection, footpad thicknesses were measured with a spring-regulated engineer's micrometer. After measurement, mice were sacrificed and specimens of the right hind footpad tissue were fixed in 10% buffered formalin for at least 48 hours and then prepared for microscopic assessment of infiltrating cells using standard techniques of morphometry performed on paraffin-embedded and hematoxylin- and eosin-stained sections. Using an ocular grid, specimens were examined in a coded fashion, and all morphologically-identified inflammatory cells were quantified.

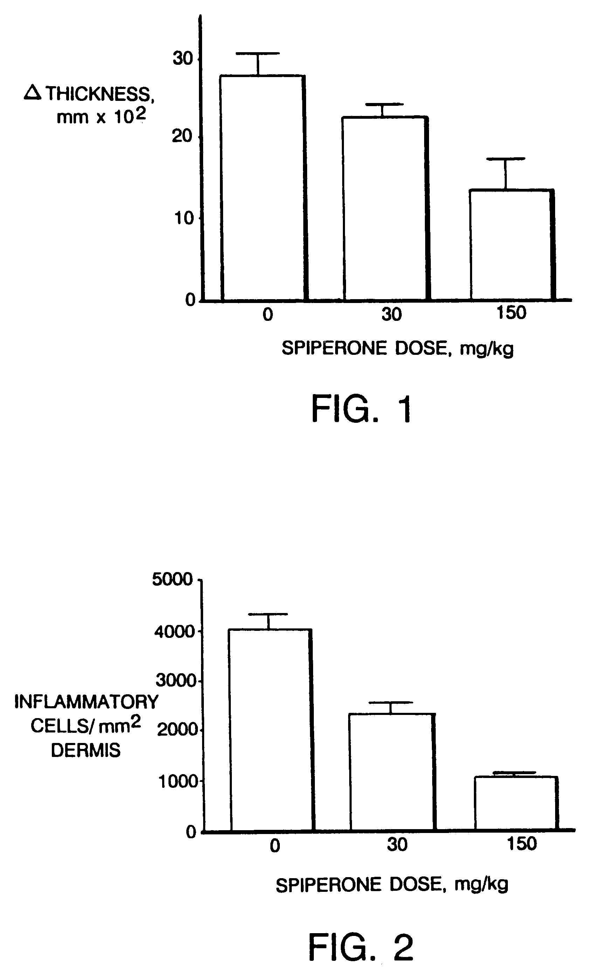

Systemic treatment with spiperone reduces the extent of rIL-1-induced inflammation, whether such inflammation is expressed as the difference ("△ thickness") between measured thicknesses of the right and left footpad of each mouse (Fig. 1), or by the degree of infiltration of inflammatory cells (Fig. 2). The △ thickness after treatment with 30 mg/kg and 150 mg/kg spiperone was approximately 10% and 50% lower, respectively, than the △ thickness seen with the untreated controls (Fig. 1), while the number of inflammatory cells per mm² of biopsied footpad tissue decreased by approximately 40% and 75% at the two respective dosage levels, compared to the untreated controls (Fig. 2).

### Example 2: Inhibition of PMA-induced Inflammation

10µl of phorbol-12-myristate-13-acetate ("PMA", Sigma Chemical Co.), dissolved in acetone at 1 mg/kg acetone, was applied to the inner surface of the right ear of each of 15 Balb/c mice. At one hour after the PMA application, mice were treated systemically with spiperone (1) or carrier (Cremophor EL) as described in Example 1. Immediately before and at 18 hours after application of PMA, ear thicknesses were measured with a spring-loaded engineer's micrometer. "△ Thickness" for ear swelling measurements represents the 18-hour value minus the baseline value. The mice were then sacrificed, and specimens of ear tissue were fixed in a fixative solution consisting of 2.0% paraformaldehyde, 2.5% glutaraldehyde, and 0.025% CaCl₂ in 0.1M sodium cacodylate buffer, pH 7.4, for 24 hours at 4°C. The specimens were stored in 0.1M sodium cacodylate buffer, pH 7.4, for an additional 24 hours, after which the old buffer was removed and fresh cacodylate buffer was added. Specimens were then prepared for microscopic quantification of morphologically-identified infiltrating inflammatory cells using standard techniques of morphometry performed on plastic-embedded and Giemsa-stained 1µm sections.

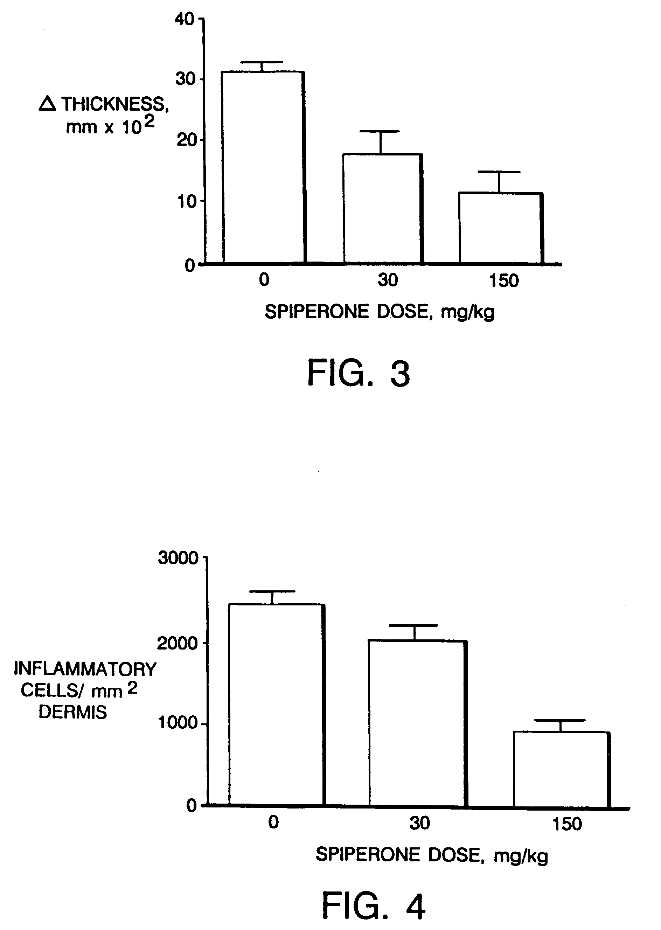

As shown in Fig. 3, spiperone at systemic doses of 30 mg/kg and 150 mg/kg is capable of reducing ear swelling related to PMA-induced inflammation by approximately 40% and 60%, respectively, compared to ear swelling observed in the controls which did not receive spiperone. In addition, inflammatory cell infiltration was decreased by about 20% and 65% in the ear tissues of mice treated with 30 mg/kg and 150 mg/kg spiperone, respectively (Fig. 4).

### Example 3: Inhibition of Oxazolone-induced Contact Hypersensitivity

The abdomens of 15 Balb/c mice were shaved with electric clippers. 50µl of 4% w/w of oxazolone (Sigma Chemical Co.) in a 4:1 v:v acetone:olive oil solution was applied to the shaved abdomen and 5µl was applied to each hind footpad of each mouse. Five to seven days later, mice were challenged for contact hypersensitivity to oxazolone by applying 10µl of the 0.4% oxazolone solution to both the inner and the outer surfaces of the right ear of each mouse. At one hour after the oxazolone challenge, mice were treated systemically with spiperone (1) in carrier (Cremophor EL), or carrier alone, as described in Example 1. Immediately before and at 24 hours after the oxazolone challenge, ear thicknesses were determined with an engineer's micrometer to compute △ thickness, as described in Example 2. Mice were sacrificed after measurements were obtained, and ear tissue was processed for determination of infiltrating inflammatory cells as described in Example 2.

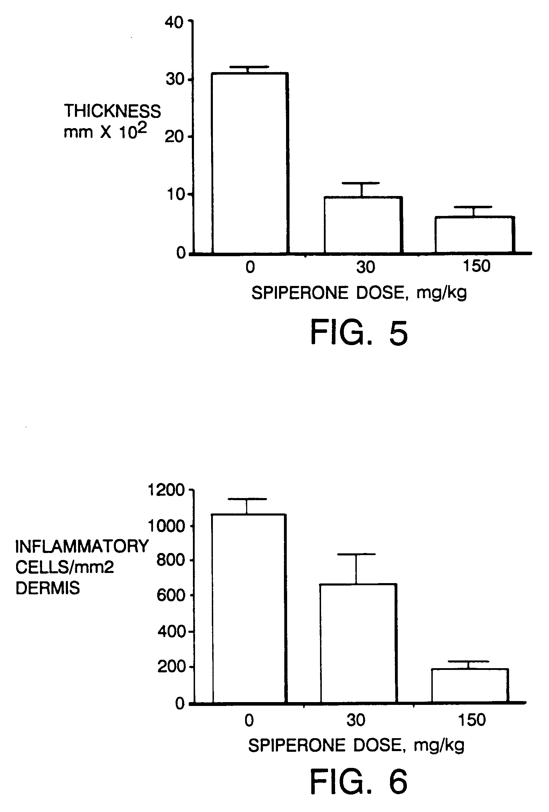

As shown in Fig. 5, spiperone is capable of inhibiting ear swelling related to oxazolone-induced cutaneous contact hypersensitivity by approximately 68% and 80% at systemic spiperone doses of 30 mg/kg and 150 mg/kg, respectively, compared to the degree of swelling exhibited by untreated control mice. The same dosages of spiperone inhibit inflammatory cell infiltration in oxazolone-challenged mice by approximately 35% and 80%, respectively, compared to the control mice which received no spiperone (Fig. 6).

Other embodiments are within the following claims.

## Claims

1. The use of spiperone or a spiperone derivative of the structure: 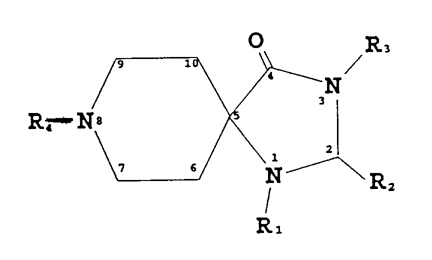 wherein
R₁ = H, CH₃-, C₆H₅-, cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)-, or (CH₃)₃C-;
R₂ = H or CH₃;
R₃ = H, CH₃, CH₃CH₂-, CH₃CH₂CH₂-, (CH₃)₂CH-, or CN(CH)₂-;
R₄ = H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃(CH₂)₃, 4-X-C₆H₄CH(CH₂CH₃)CH₂-, 4-X-C₆H₄CH(CH₃) (CH₂)₂-, 4-X-C₆H₄-CH(CH₃) (CH₂)₃-, C₆H₅CH(OCH₃)(CH₂)₂-, 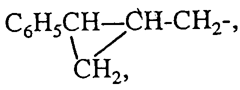 C₆H₅CO(CH₂)₄-, 4-(CH₃)C₆H₄CO(CH₂)₃-, 4-(CH₃O)C₆H₄CO(CH₂)₃, 4-X-C₆H₄CO(CH₂)₃-, 4-X-C₆H₄CO(CH₂)₃-, 2-thienyl-CO(CH₂)₃-, 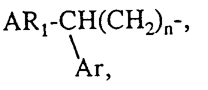 wherein n = 3 or 4; X = is F, Cl, or Br, and each of Ar and Ar₁ is, independently, H, C₆H₅-, 4-(CH₃)C₆H₄-, 4-(CH₃O)C₆H₄-, 4-X-C₆H₄-, 3-(CX₃)C₆H₄-, 2-thienyl, or 4-X-C₆H₄CH₂-, or its pharmaceutically acceptable salt, in the manufacture of a medicament for systemic administration for treating a mammal in need of immunosuppression as a result of an immune disorder.

2. The use of Claim 1 wherein the mammal is a human.

3. The use of Claim 1 or 2, wherein the immune disorder is selected from the group consisting of autoimmune diseases, diseases of unknown etiology having an immunological component, and allergies.

4. The use of Claim 3, wherein the immune disorder is selected from a group consisting of rheumatoid arthritis, juvenile rheumatoid arthritis, psoriatic arthritis, psoriasis, leprosy reversal reactions, erythema nodosum leprosum, autoimmune uveitis, multiple sclerosis, allergic encephalomyelitis, systemic lupus erythematosis, acute necrotizing hemorrhagic encephalopathy, idiopathic bilateral progressive sensorineural hearing loss, aplastic anemia, pure red cell anemia, idiopathic thrombocytopenia, polychondritis, scleroderma, Wegener's granulomatosis, chronic active hepatitis, myasthenia gravis, atopic dermatitis, Stevens-Johnson syndrome, idiopathic sprue, Crohn's disease, Graves opthalmopathy, sarcoidosis, primary biliary cirrhosis, primary juvenile diabetes, uveitis posterior, and interstitial lung fibrosis.

5. The use of any one of Claims 1 to 4, wherein spiperone or the spiperone derivative is administered in a time release formulation.

6. The use of any one of the preceding claims, wherein spiperone or the spiperone derivative is a cycloamylose complex.

7. The use of any one of the preceding claims, wherein spiperone or the spiperone derivative is administered as a pharmaceutically acceptable salt.

8. The use of any one of the preceding claims, wherein the spiperone derivative or pharmaceutically acceptable salt of spiperone or the spiperone derivative is without significant neuroleptic effect.

## Patentansprüche

1. Verwendung von Spiperon oder eines Spiperonderivats der Struktur: 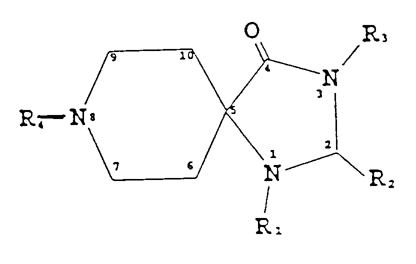 worin bedeuten:
R₁ H, CH₃-, C₆H₅-, Cyclohexyl, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)- oder (CH₃)₃C-;
R₂ H oder CH₃;
R₃ H, CH₃, CH₃CH₂-, CH₃CH₂CH₂-, (CH₃)₂CH- oder CN(CH₂)₂-;
R₄ H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃(CH₂)₃, 4-X-C₆H₄CH(CH₂CH₃)CH₂-, 4-X-C₆H₄CH(CH₃) (CH₂)₂-, 4-X-C₆H₄-CH(CH₃) (CH₂)₃-, C₆H₅CH(OCH₃)(CH₂)₂-, 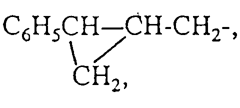 C₆H₅CO(CH)₂)₄-, 4-(CH₃)C₆H₄CO(CH₂)₃-, 4-(CH₃O)C₆H₄CO(CH₂)₃-, 4-X-C₆H₄CO(CH₂)₃-, 4-X-C₆H₄CO(CH₂)₃-, 2-Thienyl-CO(CH₂)₃-, 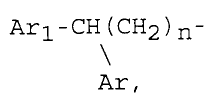 mit n = 3 oder 4; X = F, Cl oder Br, und jedem Rest Ar und Ar₁ unabhängig voneinander gleich H, C₆H₅-, 4-(CH₃)C₆H₄-, 4-(CH₃O)C₆H₄-, 4-X-C₆H₄-, 3-(CX₃)C₆H₄-, 2-Thienyl oder 4-X-C₆H₄CH₂-,
oder eines pharmazeutisch akzeptablen Salzes hiervon bei der Herstellung eines Medikaments zur systemischen Verabreichung bei der Behandlung eines Säugetiers mit Bedarf nach einer Immunsuppression als Ergebnis einer Immunstörung.

2. Verwendung nach Anspruch 1, wobei das Säugetier aus einem Menschen besteht.

3. Verwendung nach Anspruch 1 oder 2, wobei die Immunstörung aus der Gruppe Autoimmunkrankheiten, Krankheiten unbekannter Ätiologie mit einer immunologischen Komponente und Allergien ausgewählt ist.

4. Verwendung nach Anspruch 3, wobei die Immunstörung aus einer Gruppe, bestehend aus rheumatoider Arthritis, juveniler rheumatoider Arthritis, psoriatischer Arthritis, Psoriasis, Lepraumkehrreaktionen, Erythema nodosum leprosum, Autoimmunuveitis, multipler Sklerose, allergischer Enzephalomyelitis, systemischem Lupus erythematode, akuter nekrotisierender hämorrhagischer Enzephalopathie, idiopathischem beidseitigem fortschreitendem sensoneuralem Gehörverlust, aplastischer Anämie, reiner Erythrozytenanämie, idiopathischer Thrombozytopenie, Polychondritis, Sklerodermie, Wegenerscher Granulomatose, chronischer aktiver Hepatitis, Myasthenia gravis, konstitutionellem Ekzem, Stevens-Johnson-Syndrom, idiopathischer Sprue, Morbus Crohn, Basedow-Krankheit, Sarkoidose, primär-biliärer Zirrhose, primärem juvenilem Diabetes, Uveitis posterior und interstitieller Lungenfibrose, ausgewählt ist.

5. Verwendung nach einem der Ansprüche 1 bis 4, wobei das Spiperon oder Spiperonderivat in Form einer zeitgesteuerten Freigaberezeptur verabreicht wird.

6. Verwendung nach einem der vorhergehenden Ansprüche, wobei das Spiperon oder Spiperonderivat aus einem Cycloamylosekomplex besteht.

7. Verwendung nach einem der vorhergehenden Ansprüche, wobei das Spiperon oder Spiperonderivat als pharmazeutisch akzeptables Salz verabreicht wird.

8. Verwendung nach einem der vorhergehenden Ansprüche, wobei das Spiperonderivat oder pharmazeutisch akzeptable Salz des Spiperons oder des Spiperonderivats ohne signifikante neuroleptische Wirkung ist.

## Revendications

1. Utilisation d'une spipérone ou d'un dérivé de spipérone de structure : 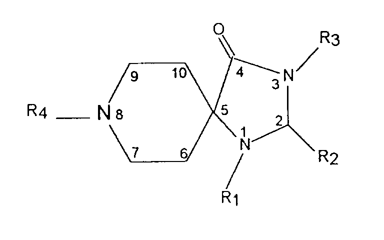 dans laquelle :
R₁ = H, CH₃-, C₆H₅-, cyclohexyle, 4-(OCH₃)C₆H₄-, 3-(CH₃)C₆H₄-, 4-(CH₃)C₆H₄-, 4-X-C₆H₄-, (CH₃)₂CH-, CH₃(CH₂)₃-, (CH₃)₂CHCH₂-, CH₃CH₂CH(CH₃)- ou (CH₃)₃C-;
R₂ = H ou CH₃;
R₃ = H, CH₃, CH₃CH₂-, CH₃CH₂CH₂-, (CH₃)₂CH- ou CN(CH₂)₂;
R₄ = H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, 4-CH₃C₆H₄CH(CH₃)(CH₂)₃-, 4-(CH₃O)C₆H₄CH(CH₃(CH₂)₃, 4-X-C₆H₄CH(CH₂CH₃)CH₂-, 4-X-C₆H₄CH(CH₃) (CH₂)₂-, 4-X-C₆H₄CH(CH₃) (CH₂)₃-, C₆H₅CH(OCH₃)(CH₂)₂-, 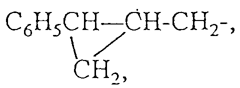 C₆H₅CO(CH₂)₄-, 4-(CH₃)C₆H₄CO(CH₂)₃-, 4-(CH₃O)C₆H₄CO(CH₂)₃, 4-X-C₆H₄CO(CH₂)₃-, 4-X-C₆H₄CO(CH₂)₃-, 2-thiényl-CO(CH₂)₃-, 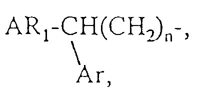 où n = 3 ou 4 ; X = F, Cl ou Br, et chacun parmi Ar et Ar₁ est indépendamment H, C₆H₅-, 4-(CH₃)C₆H₄-, 4-(CH₃O)C₆H₄-, 4-X-C₆H₄-, 3-(CX₃)C₆H₄-, 2-thiényle ou 4-X-C₆H₄CH₂-, ou de son sel pharmaceutiquement acceptable, dans la fabrication d'un médicament pour l'administration systémique pour traiter un mammifère ayant besoin d'une immunosuppression en conséquence d'un trouble immunitaire.

2. Utilisation selon la revendication 1, dans laquelle le mammifère est un humain.

3. Utilisation selon la revendication 1 ou 2, dans laquelle le trouble immunitaire est choisi dans le groupe constitué par les maladies auto-immunes, les maladies d'étiologie inconnue ayant un composant immunitaire, et les allergies.

4. Utilisation selon la revendication 3, dans laquelle le trouble immunitaire est choisi dans le groupe constitué par la polyarthrite rhumatoïde, la polyarthrite rhumatoïde juvénile, la polyarthrite psoriasique, le psoriasis, les réactions de réversion de la lèpre, l'érythème noueux lépreux, l'uvéite autoimmune, la sclérose en plaques, l'encéphalomyélite allergique, le lupus érythémateux systémique, l'encéphalopathie hémorragique nécrosante aiguë, la perte d'audition neurosensorielle bilatérale progressive idiopathique, l'anémie aplasique, l'anémie érythrocytaire pure, la thrombocytopénie idiopathique, la polychondrite, la sclérodermie, la granulomatose de Wegener, l'hépatite chronique active, la myasthénie grave, l'eczéma atopique, le syndrome de Stevens-Johnson, la sprue idiopathique, la maladie de Crohn, l'ophtalmopathie de Graves, la sarcoïdose, la cirrhose biliaire primitive, le diabète juvénile primitif, l'uvéite postérieure et la fibrose interstitielle pulmonaire.

5. Utilisation selon l'une quelconque des revendications 1 à 4, dans laquelle la spipérone ou le dérivé de spipérone est administré(e) dans une formulation à libération retard.

6. Utilisation selon l'une quelconque des revendications précédentes, dans laquelle la spiperone ou le dérivé de spipérone est un complexe de cycloamylose.

7. Utilisation selon l'une quelconque des revendications précédentes, dans laquelle la spipérone ou le dérivé de spipérone est administré(e) sous la forme d'un sel pharmaceutiquement acceptable.

8. Utilisation selon l'une quelconque des revendications précédentes, dans laquelle le dérivé de spipérone ou le sel pharmaceutiquement acceptable de spipérone ou du dérivé de spipérone est sans effet neuroleptique significatif.
